# EUROPEAN PATENT APPLICATION

(11) **EP 2 712 606 A2**
(43) Date of publication of application: **02.04.2014**
(21) Application number: 13182418.7
(22) Date of filing: 30.08.2013
(51) Int. Cl.: A61K 8/31, A61Q 9/02, A61K 8/92, A61K 8/97, A45D 27/00, B05D 5/00

(54) **Razor blade preservation system and method**

(30) Priority: 31.08.2012 US 201261695680 P
(71) Applicant: ABJP, LLP, Boca Raton, FL 33496 (US)
(72) Inventor: PISICCHIO, Joseph, Boca Raton, FL Florida 33496 (US)
(74) Representative: Melin Granberg, Linda

(57) **Abstract**

A composition for extending the usable life of a razor blade includes a vegetable oil, for example sunflower oil, Aloe Vera gel, and Petrolatum. The composition is used by application of one to several drops of the composition to a razor blade after use followed by storage of the razor blade in the normal manner.

## Description

### BACKGROUND

The present invention relates generally to a composition and method for extending the useful life of a razor blade. More particularly the invention relates to a composition that can be applied to a razor blade between uses that does not require soaking the blade in a solution.

One of the drawbacks in the use of razors is the relatively short useable life of razor blades. Use and storage of blades in a moist environment can cause corrosion of the blade surface, especially the cutting surface, for example by oxidation, corrosion, rust, and calcification. Corrosion causes physical damage to the cutting surface of the blade so that it becomes rough and loses the sharp cutting edge. As a result of this corrosion and physical change to the blade surface, use of the blade results in discomfort when shaving by causing drag on the skin and loss of a clean cutting surface. The user is thus likely to replace the blade which can result in considerable expense over time. In fact, manufacturers of razors and blades tend to sell many blades for each razor that is sold.

Modem razor blades have come to incorporate various devices in order to provide additional comfort to users. For example, some blades add a lubricating strip to assist in smoothness when shaving. Other blades include a coating on the blade surface to reduce drag and extend blade life. A lubricating strip can be a polyurethane or other similar material that is impregnated with, for example, acrylic polymers. These strips are mounted on the head of the razor in front of the blades. The polymer film absorbs water and becomes very slippery, thus creating a lubricating surface that helps reduce drag and allow the blade to glide more smoothly across the surface of the skin.

There have been a number of compositions and methods proposed to prevent corrosion and extend the useful life of blades. Most of these methods involve placing the blade in a liquid composition or solution between uses, usually after some washing and/or drying of the blade to prevent water and air from reaching the blade. (See, for example, GB555,655; U.S. Patent Nos. 6,257,247; 6,039,937; 3,808,920; 5,958,394; 7,858,027; and 6,006,760; U.S. Patent App. Publ. Nos. 2011/0138638; 2009/0188816; and 2004/0235692.) These methods suffer from several drawbacks. For example, storage of a razor or blade in a solution requires use of a receptacle for storing the blade which is inconvenient for the user and unduly takes up space. In an attempt to alleviate or reduce this problem, numerous receptacles for storing razors and blades have been developed. (See, for example, U.S. Patent Nos. 6,257,247; 7,858,027; 5,934,459; 5,251,752; 6,006,760; and 7,559,330; U.S. Patent App. Publ. Nos. 2011/0138638; 2009/0188816; and 2011/0225833). None of these solve the problem of requiring storage space, however. Furthermore, when razors or blades are removed from the storage solution, the blades are wet and can undesirably result in dripping of the solution from the blade. In addition, because these solutions are often left open to the air, the components can evaporate resulting in inconsistency of results or deterioration of the capacity to provide protection against corrosion of the blade. Also, because the razor is repeatedly stored in the same solution or receptacle, hair and other debris that remains on the blade after use, even with rinsing, accumulates in the storage solution so that it must frequently be replaced.

Another drawback to existing storage solutions is reactivity with lubricating strips on blades. The storage solutions can swell, degrade or deteriorate the material or contents of the lubricating strips so that they do not function as intended and, in fact encumber use of the blade resulting in disposal and the inconvenience and expense of changing the blade even more often.

There thus remains a need for simple easy to use compositions and methods that can protect razor blades from corrosion and extend the usable life of the blade.

### BRIEF SUMMARY OF THE INVENTION

In summary, the invention is a composition and method that can be used to extend the usable life of razor blades.

This invention succeeds where previous efforts have failed by providing an easy to use method that does not require additional storage space. The compositions do not cause swelling or deterioration of lubricating strips of modem razor blades. The present invention succeeds where others have failed by providing a system whereby razors or blades can be stored in a normal manner after use without deterioration or corrosion.

The invention is a composition for extending the usable life of a razor blade that includes an oil, Aloe Vera gel and Petrolatum. In exemplary embodiments, the oil is a vegetable oil, for example sunflower oil. In embodiments, at least about 98% of the composition is vegetable oil, Aloe Vera gel and Petrolatum. The composition can also include Aloe concentrate, for example in an amount of from about 0.5% to about 1.5%. In embodiments, at least about 99% of the composition is vegetable oil, Aloe Vera gel, Petrolatum and Aloe concetrate. Compositions according to the invention can also include one or more of Vitamin E, Vitamin D, Vitamin A, retinyl palmitate, BHT, Tea Tree Oil and Jojoba oil. An exemplary composition includes about 85% to about 95% of a vegetable oil, for example sunflower oil, about 2.5% to about 7.5% petrolatum, and about 2.5% to about 7.5% Aloe Vera Gel. Exemplary compositions can also include one or more components such as from about 0.05% to about 0.5% Vitamin E, from about 0.05% to about 0.5% Vitamin D, from about 0.05% to about 0.5% Vitamin A or retinyl palmitate, about 0.5% to about 2.0% Aloe concentrate or from about 0.01 % to about 0.1 % BHT. Exemplary compositons can include about 89.65 % Sunflower Oil, about 5.00 % Petrolatum, about 5.00 % Aloe Vera Gel, about 0.10 % Vitamin-D, about 0.10 % Vitamin-E, about 0.10 % retinyl palmitate, about 1.00 % Aloe Concentrate, and about 0.05 % BHT.

The invention is also a method of extending the usable life of a razor blade that includes the step applying the composition according to the invention to a razor blade after use. In some embodiments, the composition is applied by placing about one to three drops of the composition directly on the blade; by applying the composition to a sponge, brush, fabric, applicator or other substrate and swiping the composition onto the blade; or by dipping the blade into the composition. In exemplary embodiments, the blade is not stored by soaking in the composition between uses. The method can also include at least partially drying the razor blade, for example by shaking excess water off of the blade. The razor blade can be used a second time without rinsing or removal of the composition.

Further objectives and advantages, as well as the structure and function of preferred embodiments will become apparent from a consideration of the description, drawings, and examples.

### BRIEF DESCRIPTION OF THE DRAWINGS

The foregoing and other features and advantages of the invention will be apparent from the following, more particular description of a preferred embodiment of the invention, as illustrated in the accompanying drawings.

FIG. 1 depicts an untreated disposable razor used one (1) month (top) in comparison with a razor of the same type that was treated with a composition according to the invention after each use for two months (bottom);

FIG. 2 depicts a second type of razor used for six (6) months that was treated with a composition according to the invention after each use; FIG. 3 depicts a comparison of the razor used for six (6) months with treatment of the invention (top) (previously shown in FIG. 2) to a razor of the same type used three (3) months without treatment (bottom).

FIG. 4 illustrates untreated razor blades after one hour in a salt spray chamber showing the resultant corrosion. FIGS. 4A and 4B are Schick Xtreme3® razors and FIGS. 4C and 4D are Gillette Fusion® razor cartridges.

FIG. 5 illustrates razor blades treated according to the invention after one hour in a salt spray chamber and showing a lack of corrosion. FIGS. 5A and 5B are Schick Xtreme3® razors and FIGS. 5C and 5D are Gillette Fusion® razor cartridges.

FIG. 6 illustrates untreated razor blades after four hours in a salt spray chamber showing the resultant corrosion. FIGS. 6A and 6B are Schick Xtreme3® razors and FIGS. 6C and 6D are Gillette Fusion® razor cartridges.

FIG. 7 illustrates razor blades treated according to the invention after four hours in a salt spray chamber and showing a lack of the formation of corrosion. FIGS. 7A and 7B are Schick Xtreme3® razors and FIGS. 7C and 7D are Gillette Fusion® razor cartridges.

FIG. 8 illustrates an untreated Gillette Fusion® razor after seven days of use. Figure 8A is an EDS analysis of the base material of the blade; FIG. 8B is a photograph of the blade surface; FIG. 8C is a scanning electron micrograph of the surface of the blade; and FIG. 8D is an EDS analysis of the material deposited on the blade.

FIG. 9 illustrates a Gillette Fusion® razor after thirty days of use with treatment according to the invention. FIG. 9A is a photograph of the blade surface; FIG. 9B is a scanning electron micrograph of the surface of the blade; and FIG. 9C is an EDS analysis of the material deposited on the blade.

### DETAILED DESCRIPTION OF THE INVENTION

Embodiments of the invention are discussed in detail below. In describing embodiments, specific terminology is employed for the sake of clarity. However, the invention is not intended to be limited to the specific terminology so selected. While specific exemplary embodiments are discussed, it should be understood that this is done for illustration purposes only. A person skilled in the relevant art will recognize that other components and configurations can be used without parting from the spirit and scope of the invention. All references cited herein are incorporated by reference as if each had been individually incorporated.

Unless indicated otherwise, percentages (%) are percentages by weight. The term "about" is generally intended to provide a variance of plus or minus 10%, as well as the number provided. Thus, about 10% is intended to include amounts from 9% to 11 % as well as 10%, and about 50% is intended to include from 45% to 55%, as well as 50%.

In embodiments, the present invention provides a composition that includes an oil, for example a vegetable oil, petrolatum and Aloe Vera gel. Other components can be present that provide further lubrication or soothing properties. For example, the formulation can include one or more of Vitamin E, Vitamin D, Vitamin A, retinyl palmitate, Aloe concentrate, menthol, fragrances and the like, as well as other cosmetic antioxidants such as BHT (butylated hydroxytoluene), Tea Tree Oil or Jojoba Oil. The formulation may also include natural or synthetic anti-microbial components.

The method of using the formulation involves, after shaving, optionally tapping the razor to remove excess water and applying the composition to the razor blade. The composition can be applied using an eyedropper, pump, or similar device to apply one to three drops to the blade surface. Alternatively, the composition can be applied to a sponge, brush, fabric or other substrate and then at least a portion of the composition transferred to the razor blade by swiping, rubbing or wiping. The sponge, brush, fabric or other substrate can be part of an applicator. Suitable applicators include, for example, brushes such as those used for applying nail polish, lip gloss and the like; doe foot applicators; cosmetic sponges; and others. The razor blade can also be dipped in the composition, and usually removed. After application of the composition to the blade, the razor is stored in the normal way, for example in a medicine cabinet. Soaking of the blade in the composition is not generally required and in most embodiments the razor is stored without soaking between uses. The composition has sufficient viscosity and adhesion properties so that leakage from the blade during storage is insubstantial. Furthermore, when the blade is retrieved for use, there is very little or no dripping of any residual composition from the blade and the problems encountered with common storage solutions is avoided. In most embodiments, the razor blade is not stored in the composition between uses.

The composition can be applied using any suitable method. For example, the composition can be stored in a bottle and applied with an eyedropper, dispensed from a container directly onto the razor blade, or dispensed from the container onto a substrate such as a sponge or cloth and applied to the blade contacting the blade with the substrate, for example by wiping, swiping or rubbing the razor blade, in order to transfer at least a portion of the composition to the razor blade. Alternatively, the composition can be applied to an applicator such as a brush or doe foot applicator, for example by dipping the applicator into the composition, and contacting the applicator with the razor blade, for example by wiping, swiping, brushing or rubbing, to apply at least a portion of the composition to the razor blade. In yet another alternative embodiments, the composition can be placed in a dispensing container. For example, the composition can be placed in a pump container and applied by pumping, for example by pumping dropwise, onto the razor blade. An exemplary pump container is an airless pump container such as manufactured by Mega Pump. Other suitable containers and dispensing containers such as dropper bottles can also be used to store and or dispense an/or apply the composition.

Compositions according to the present invention are formulated to provide sufficient viscosity and suitable rheological and adhesion properties to allow the composition to be conveniently applied to the blade and to remain adhered to the blade without leakage or dripping during storage. Prior art formulations designed primarily as a storage solution lack the viscosity and adhesion properties necessary to prevent such leakage. In fact, by design, such prior storage solutions should not be too viscous so that when the blade is removed from the solution the bulk of the solution drips from the blade back into the storage reservoir. In contrast, the present solution is intended to remain on the blade and not to easily drip from or fall off of the blade. In use as described above, the composition remains on the blade during storage and need not be removed prior to reuse. By remaining on the blade, the compositions according to the invention can better exert lubricating and soothing properties inherent in the components. In exemplary embodiments, the viscosity of the composition can be at least about 500 cSt at 25°C, at least about 1000 cSt at 25°C , or at least about 1500 cSt at 25°C. In some embodiments, the viscosity is from about 500 to about 3000 cSt at 25°C, from about 1000 to about 2500 cSt at 25°C or from about 1500 to about 2200 cSt at 25°C. In exemplary embodiments, the viscosity is less than about 3000 cSt at 25°C, less than about 2500 cSt at 25°C, less than about 2200 cSt at 25°C or less than about 2000 cSt at 25°C. In exemplary embodiments, viscosity is about 2000 cSt at 25°C

Exemplary formulations of the invention include from about 75% to about 95% of an oil such as a vegetable oil. Some embodiments include from about 85% to about 95% oil, for example about 90% oil. In exemplary embodiments, 100% of the oil component of the composition is a vegetable oil. In exemplary embodiments, vegetable oils are used to provide an all-natural base to the formulation. Although a range of vegetable oils can be used, sunflower oil is an exemplary vegetable oil. Sunflower oil provides superior glide properties, and natural protection for the skin, as well as excellent coating and dispersion properties when applied to a razor blade. As an alternative to sunflower oil, other oils with similarly suitable properties can be used. Examples of other oils include olive oil and coconut oil. Other suitable oils can include safflower oil, corn oil, soybean oil, cottonseed oil, canola oil and sesame oil. The invention can also utilize mixtures of vegetable oils, for example a mixture of sunflower oil with another vegetable oil. While synthetic and silicon oils such as dimethicone can be used in place of vegetable oil, use thereof is not as desirable, although embodiments of the invention can use synthetic oils in combination with a vegetable oil. In embodiments, the amount of synthetic oils is less that the amount of vegetable oil.

Formulations according to the invention also include petrolatum. In particular, the use of petrolatum is believed to provide improved viscosity, adhesion and rheological properties to the composition so that it is better able to remain on the blade during storage and use. While such properties are advantageous to the present composition and method, adhesion and relatively high viscosity would be disadvantageous in soaking solutions as used in the prior art. The petrolatum can be of various types, but USP petrolatum, for example Petrolatum USP Jelly, is an exemplary petrolatum. In exemplary embodiments, petrolatum can be present in amounts from about 2.5% to about 25%. In some embodiments, petrolatum makes up from about 2.5% to about 10%, for example from about 2.5% to about 7.5%, or about 5% of the composition.

Prior art formulations frequently utilize isopropyl myristate (IPM) to prevent corrosion of razor blades. Initial attempts to make a composition suitable for use as described above incorporated IPM. Unfortunately, it was found that when used with coated blades or blades having lubricating strips, compositions that include IPM resulted in swelling, degradation and deterioration of the lubricating strips. When IPM was removed in order to avoid such deterioration, the properties of the composition were less desirable. For example, razor glide across the skin diminished causing more friction while shaving and the razor accumulated more build up between shaves. It was surprisingly found, however, that in suitable concentrations the use of Aloe Vera gel as a solvent-like component overcame the disadvantages caused by removal of the IPM. Aloe Vera gel has been used in soaking solutions for razor blades for its soothing and lubricating properties, but has not previously been suggested as a replacement or surrogate for IPM. Aloe Vera gel, as used in the present formulation, provides not only soothing and lubricating properties, but improves the rheological properties and viscosity of the composition. In exemplary embodiments, Aloe Vera gel can be present in amounts ranging about 2.5% to about 10%, for example from about 2.5% to about 7.5%, or about 5% of the composition.

Additional lubricating and soothing properties can be obtained with the present invention by including within the composition Aloe concentrate. Aloe concentrate includes aloe spray dried powder. The Aloe concentrate can be prepared by, for example, spray drying of whole aloe or, in exemplary embodiments, from spray drying Aloe Vera gel or the fillets of the aloe leaf. An exemplary Aloe concentrate is generally referred to as Aloe Vera spray dried powder, Aloe Vera Gel spray dried powder, Aloe Vera extract spray dried powder and the like. Aloe concentrate can be provided in a number of concentrate strengths, for example 50:1 (50X), 100:1 (100X), and 200:1 (200X). Aloe spray dried concentrate 200X is an exemplary material. The strength general refers to the amount of water required for reconstitution. For example, reconstitution of 100X Aloe concentrate is accomplished by adding one part concentrate to 99 parts water. In exemplary embodiments, the Aloe concentrate can be present in amounts up to about 2.5% of the composition, for example from about 0.1% to about 2.5%, for example from about 0.5% to about 2.0%, from about 0.5 to about 1.5% or about 1.0% of the composition.

Additional components can be present in the composition to provide further antioxidant or lubricant properties. Examples of additional components include, without limitation, Vitamin E, Vitamin D, Vitamin A, and retinyl palmitate. These components can be present, for example, in amounts up to about 2.0%, for example from about 0.05% to about 1%, from about 0.05% to about 0.5%, or about 0.1% of the formulation. Other antioxidants, such as, for example BHT and related compounds, Tea Tree Oil and Jojoba oil, can also be present in amounts up to about 2.0%, for example from about 0.01 % to about 1%, from about 0.01 % to about 0.1%, or about 0.05% of the formulation. Other components can be added to impart various beneficial or useful properties to the oil such as further antioxidants, fragrances, lubricants, antimicrobials, antibacterials, or components that affect skin feel by imparting, for example, soothing or cooling properties and the like. For example, Tea Tree Oil and/or Jojoba oil can be added in amounts up to about 0.05%, for example from about 0.005% to about 0.025%, about 0.005% to about 0.015%, or about 0.01%.

Exemplary compositions according to the present invention include about 85% to about 95% of an oil such as a vegetable oil, for example sunflower oil, about 2.5% to about 7.5% petrolatum, and about 2.5% to about 7.5% Aloe Vera Gel. Some embodiments of the invention include about 90% of a vegetable oil, for example sunflower oil, about 5% petrolatum, and about 5% Aloe Vera gel. In addition to vegetable oil, petrolatum, and Aloe Vera gel, embodiments of the invention can also include about 0.5% to about 2.0% Aloe concentrate. Some exemplary embodiments include about 1.0% Aloe concentrate.

In exemplary embodiments of the invention, about 95% to about 99% of the composition consists of vegetable oil, petrolatum and Aloe Vera gel. In exemplary embodiments of the invention, more than 99% of the composition consists of vegetable oil, petrolatum and Aloe Vera gel. In exemplary embodiments of the invention, about 95% to about 99% of the composition consists of vegetable oil, petrolatum, Aloe Vera gel, and Aloe concentrate. In exemplary embodiments of the invention, more than 99% of the composition consists of vegetable oil, petrolatum, Aloe Vera gel, and Aloe concentrate.

Some embodiments of the invention can further include one or more of from about 0.05% to about 0.5% Vitamin E, from about 0.05% to about 0.5% Vitamin D, from about 0.05% to about 0.5% Vitamin A or retinyl palmitate. Exemplary embodiments of the invention include, in addition to vegetable oil, petrolatum and Aloe Vera gel, about 0.1% Vitamin E, about 0.1 % Vitamin D, and about 0.1 % retinyl palmitate. Some embodiments of the invention can further include from about 0.01 % to about 0.1 % BHT. Exemplary embodiments include about 0.05% BHT.

In one embodiment of the invention, the composition includes about 89.65 % Sunflower Oil, about 5.00 % Petrolatum, about 5.00 % Aloe Vera Gel, about 0.10 % Vitamin-D, about 0.10 % Vitamin-E, about 0.10 % retinyl palmitate, about 1.00 % Aloe Concentrate, and about 0.05 % BHT.

### EXAMPLES

### EXAMPLE 1 - Formulations

### EXAMPLE 1A:

An exemplary formulation according to the invention contains:

| | |
|---|---|
| Sunflower Oil | 88.63 % |
| Petrolatum | 5.00 % |
| Aloe Vera Gel | 5.00 % |
| Vitamin-D | 0.10 % |
| Vitamin-E | 0.10 % |
| Retinyl Palmitate | 0.10 % |
| Aloe Concentrate 200X | 1.00 % |
| BHT | 0.05 % |
| Jojoba Oil | 0.01 % |
| Tea Tree Oil | 0.01 % |

### EXAMPLE 1B:

An exemplary formulation according to the invention contains:

| | |
|---|---|
| Sunflower Oil | 89.63 % |
| Petrolatum | 5.00 % |
| Aloe Vera Gel | 5.00 % |
| Vitamin-D | 0.10 % |
| Vitamin-E | 0.10 % |
| Retinyl Palmitate | 0.10 % |
| BHT | 0.05 % |
| Jojoba Oil | 0.01 % |
| Tea Tree Oil | 0.01 % |

### EXAMPLE 2

Two Schick disposable razors were tested for comparison. The first razor was used daily for one month under normal conditions (i.e. without any further treatment). The second razor was used daily for about two months. During the first one month period, the first razor was used to shave one half of the user's face, and the second razor to shave the other half. After each daily use, the second razor was shaken dry, and 2-3 drops of the composition of EXAMPLE 1B applied to the razor blade and lubricating strip and the razor stored on a shelf under normal conditions. The razor was not rinsed or otherwise treated before the next use.

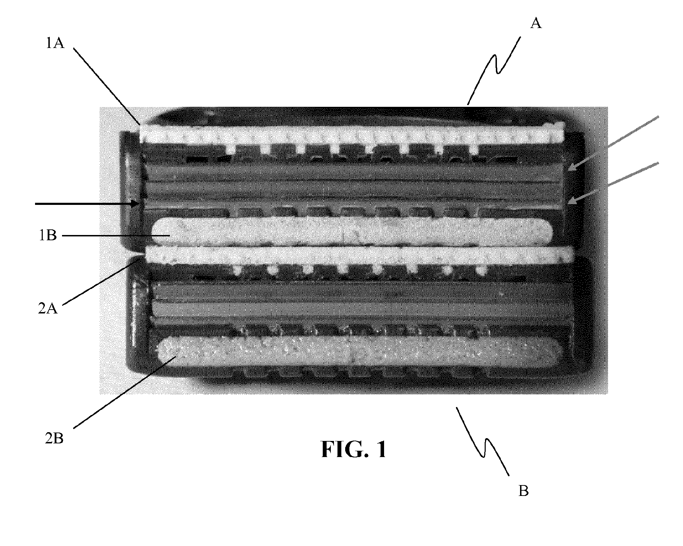

At the end of the test period, one month for the untreated razor and two months for the treated razor, the two razor were photographed. FIG. 1 depicts a comparison of the two razors showing the first untreated razor on top and the second treated razor on the bottom. As can be seen in FIG. 1, after just one month of use the untreated razor (**A**) shows signs of corrosion and rust as indicated by the arrows. In contrast, after two months of use with daily treatment by the inventive composition, the treated razor (**B**) is devoid of signs of corrosion or rust. The difference in the amount of use is apparent from the greater degree of wear on the plastic strip of the treated razor (**1B**) as compared to the degree of wear on the plastic strip of the untreated razor (**1A**). Furthermore, because the lubricating strip of the second razor was treated along with the blade, the lubricating strip of the untreated razor (**1B**) shows more wear than the lubricating strip of the treated razor (**2B**).

### EXAMPLE 3

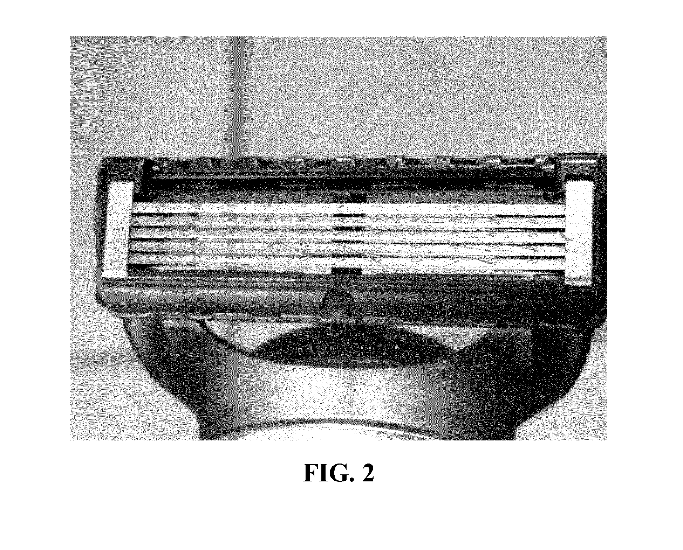

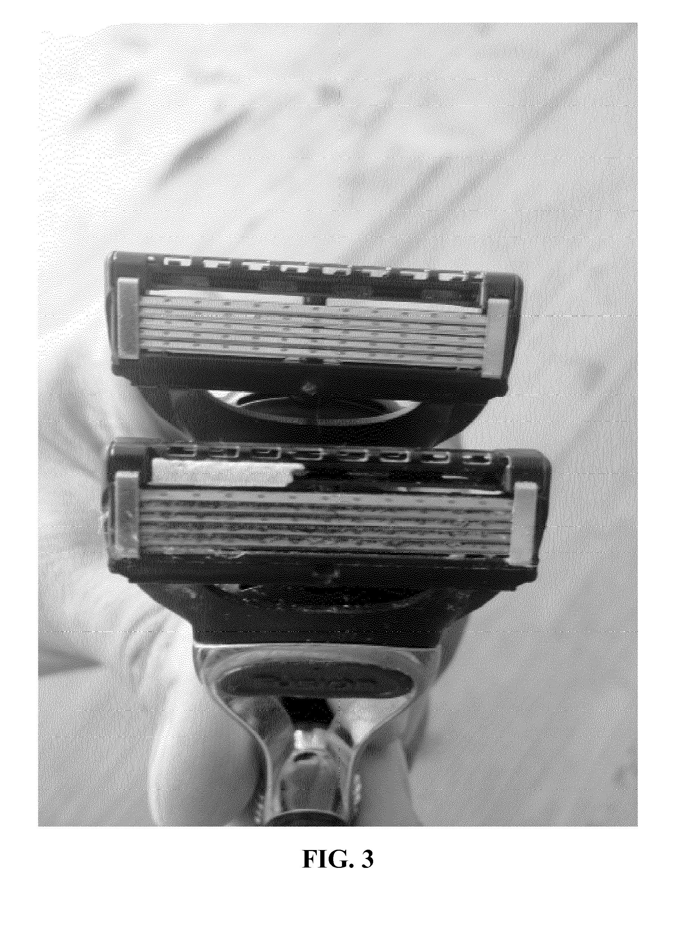

Two Gillette Fusion razors were tested for comparison. A first razor was used daily for six (6) months. After each daily use, the first razor was shaken dry, and 2-3 drops of the composition of EXAMPLE 1B applied and the razor stored on a shelf under normal conditions. The razor was not rinsed or otherwise treated before the next use. FIG. 2 is a photograph of the first razor at the end of six months. The first razor is also shown as the top razor in FIG. 3.

A second razor was used daily for three (3) months under normal conditions (i.e. without any further treatment). FIG. 3 is a photograph comparing the first and second razors. The second razor, after three months of use, is the bottom razor of FIG. 3 and is seen in comparison with the first razor, after six months of use, which is illustrated as the top razor in FIG. 3. As is readily apparent from FIG. 3, the blades of the second razor exhibit significant corrosion in the form of oxidation and calcification. In contrast, the first treated razor shows virtually no evidence of corrosion and calcification.

### EXAMPLES 4-7

Samples were tested using ASTM B117 which is an accelerated corrosion salt spray test in which samples are placed In a salt spray chamber. A typical salt spray test for passivation of 300 series stainless steel is two hours. Test times used for evaluating the present invention were one hour (EXAMPLES 4 and 5) and four hours (EXAMPLES 6 and 7).

### COMPARATIVE EXAMPLE 4

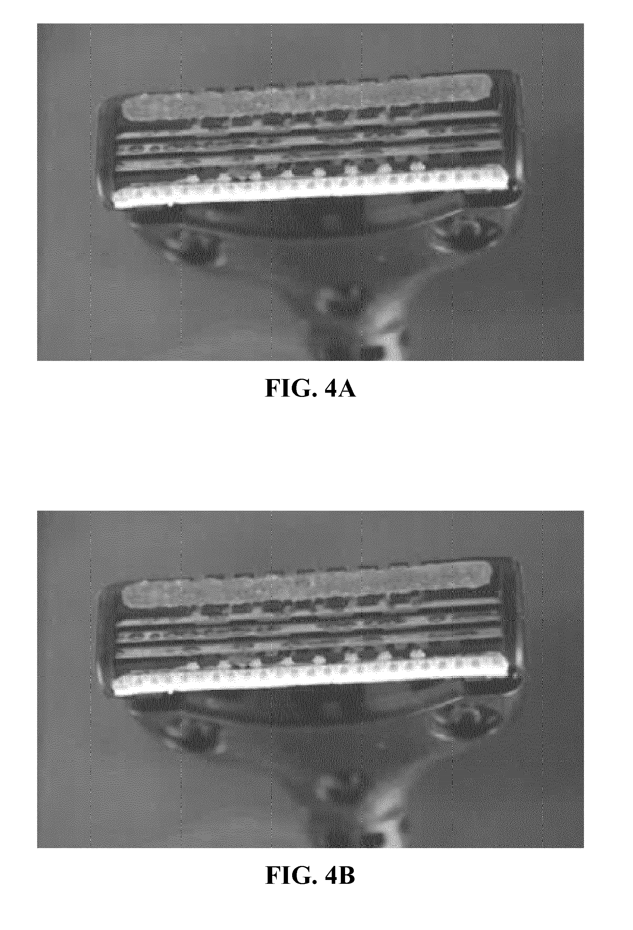

Two Schick Xtreme3® razors were placed in a salt spray chamber and removed after one hour. FIG. 4A illustrates a first razor showing rust on each of the three cutting blades. FIG. 4B illustrates a second razor showing corrosion of all three blades with close to 50% of the surface corroded.

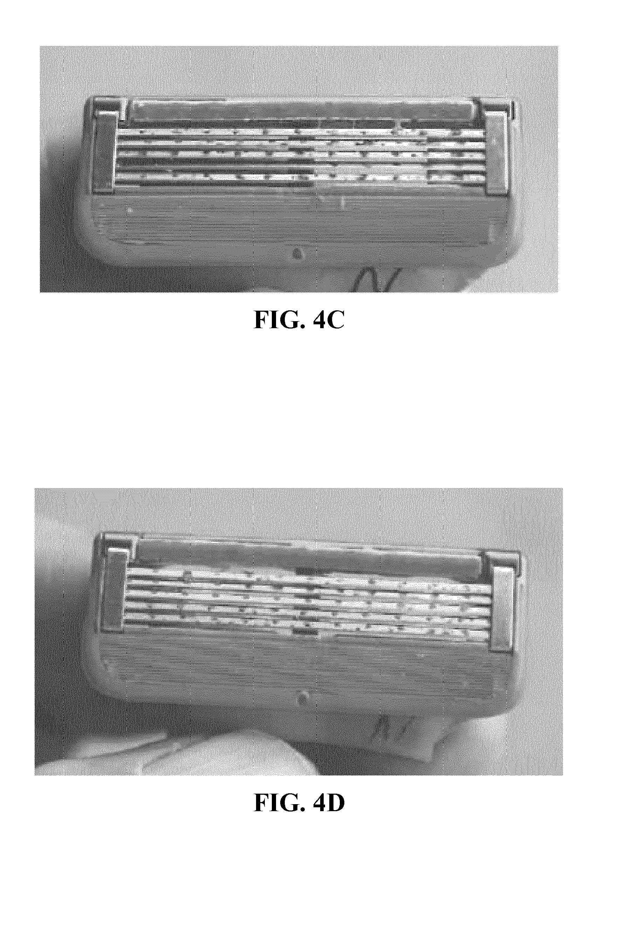

Two Gillette Fusion® razor cartridges were placed in a salt spray chamber and removed after one hour. FIG. 4C illustrates a first razor cartridge and FIG. 4D illustrates a second razor cartridge after exposure. Although less corrosion is observed than with the Schick Xtreme3® razors, some corrosion is still evident.

### INVENTIVE EXAMPLE 5

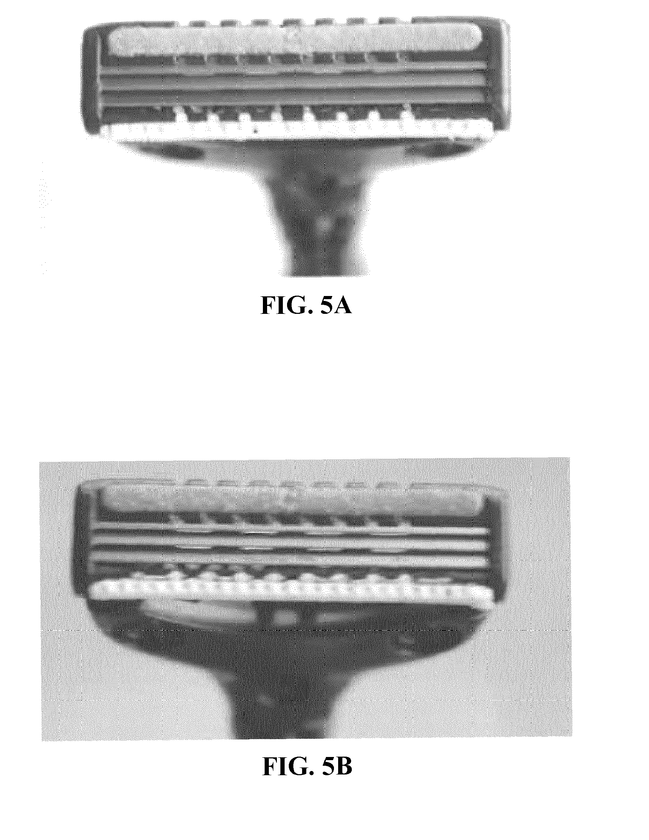

Two Schick Xtreme3® razors and two Gillette Fusion® razor cartridges were pretreated by placing several drops of the composition of EXAMPLE 1B on the razor blades. These blades and cartridges were placed in a salt spray chamber and removed after one hour. FIGS. 5A and 5B illustrate the blades of the Schick Xtreme3® razors after testing. The blade illustrated in FIG. 5A shows only very light corrosion on one of the three blades. The blade illustrated in FIG. 5B showed no indications of any oxidation.

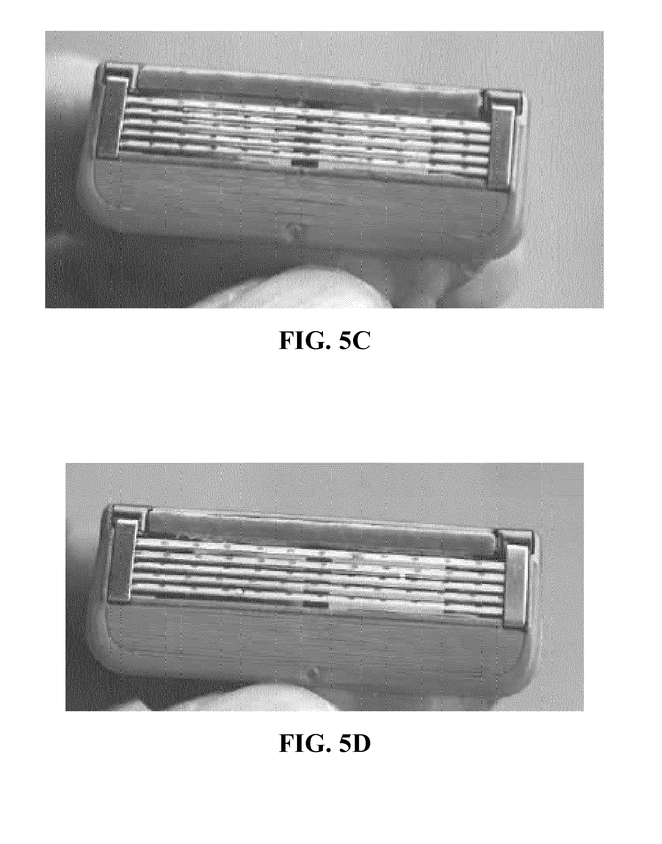

FIGS. 5C and 5D illustrate the blades of the Gillette Fusion® razor cartridges after testing. There are no signs of corrosion on any blades of either cartridge.

### COMPARATIVE EXAMPLE 6

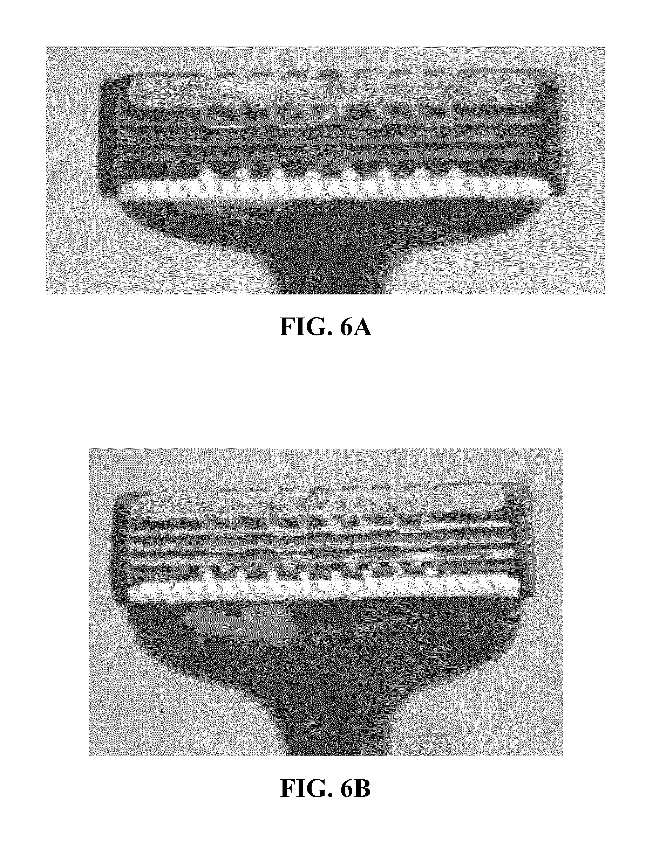

Two Schick Xtreme3® razors were placed in a salt spray chamber and removed after four hours. FIG. 6A illustrates a first razor showing that almost all the surface of the blade has exhibits some corrosion in the form of rust. FIG. 6B illustrates a second razor showing in somewhat better condition but still reveals rust at well over 50% of the blade area.

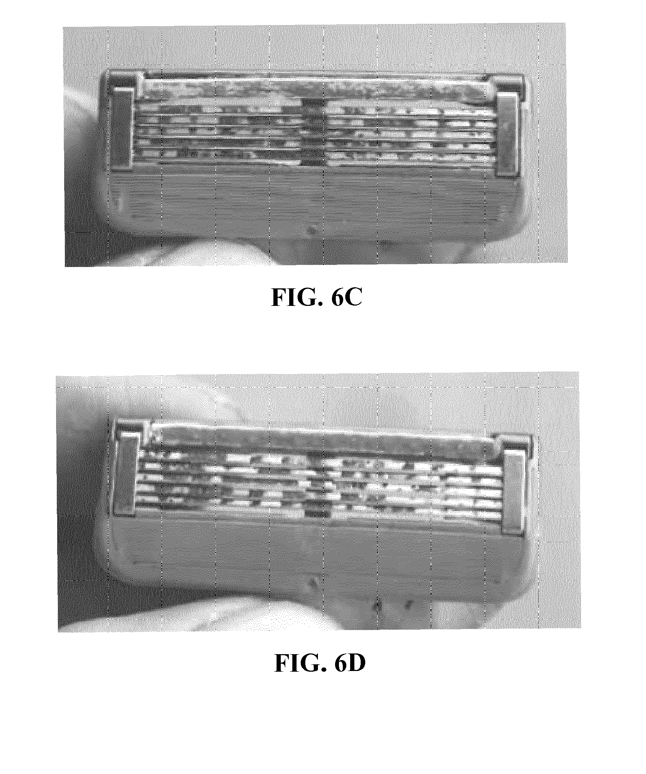

Two Gillette Fusion® razor cartridges were placed in a salt spray chamber and removed after four hours. FIGS. 6C and 6D illustrate a first and second razor cartridge. In contrast to the razor cartridges of the same type tested by one hour salt spray exposure, the subjected to four hours of exposure show substantial oxidation on the surface of the cutting blades.

### INVENTIVE EXAMPLE 7

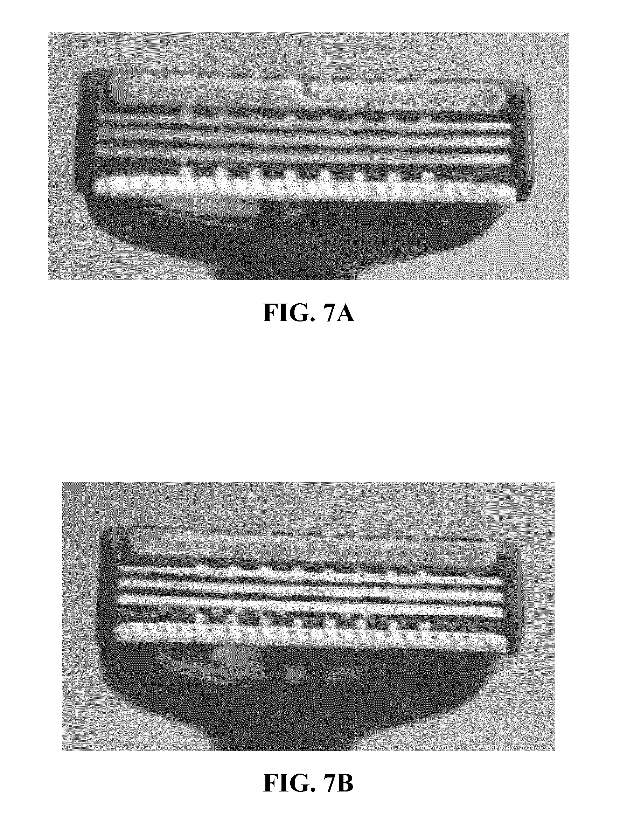

Two Schick Xtreme3® razors and two Gillette Fusion® razor cartridges were pretreated by placing several drops of the composition of EXAMPLE 1B on the razor blades. These blades and cartridges were placed in a salt spray chamber and removed after four hours. FIGS. 7A and 7B illustrate the blades of the Schick Xtreme3® razors after testing. These revealed very little to no corrosion. There is a slight amount of oxidation or staining on each of the razors but is very slight and less than what the untreated razors exhibited after just one hour of salt spray. (EXAMPLE 4)

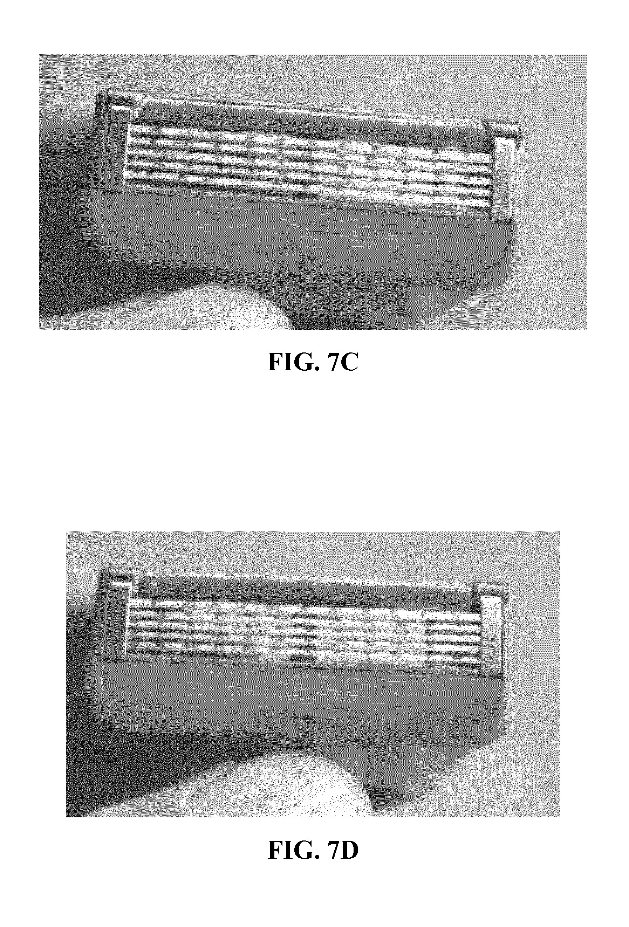

FIGS. 7C and 7D illustrate the blades of the Gillette Fusion® razor cartridges after testing. The unit shown in FIG. 7C shows a very slight area of oxidation but there was no oxidation or corrosion observed on the second sample shown in FIG. 7D. Again, the blades are in better condition than those exposed to salt spray for only one hour without treatment according to the invention. Thus, after four hours of salt spray, razor blades treated according to the composition and method of the invention were in better condition than those without treatment, indicating that treated blades are over four times more corrosion resistant than untreated blades.

### COMPARATIVE EXAMPLE 8

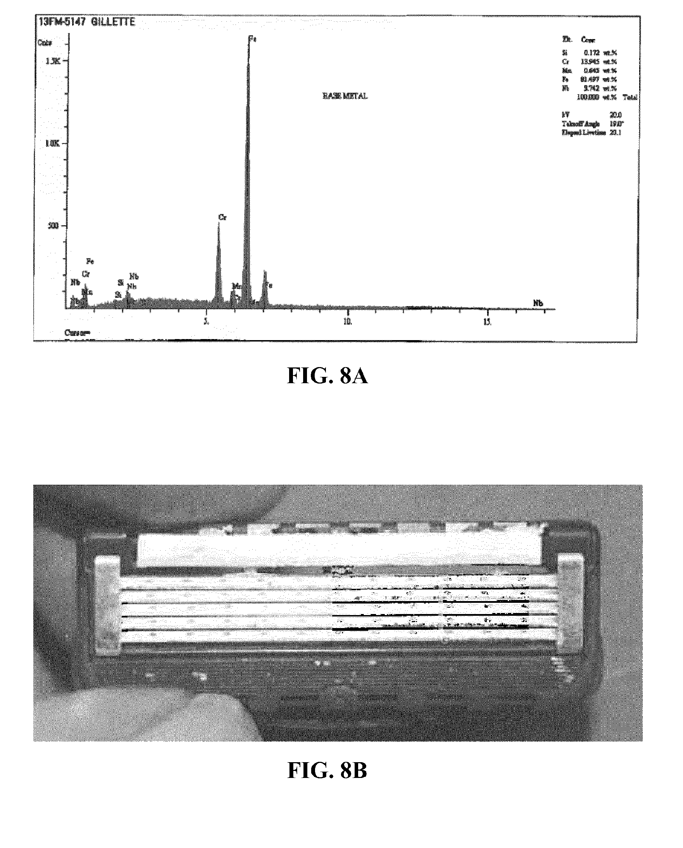

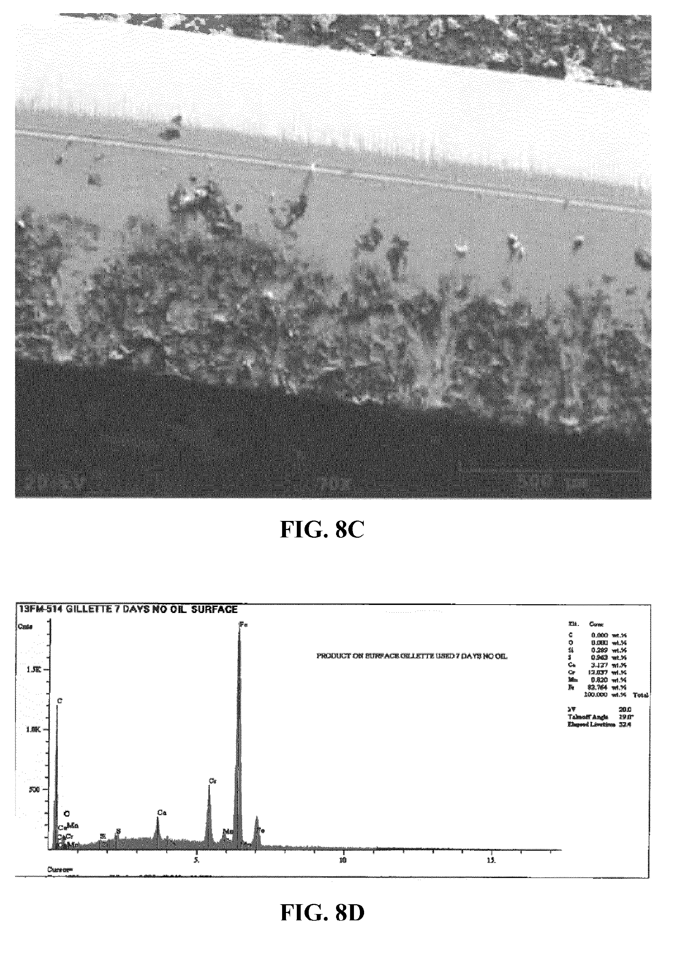

Energy-dispersive X-ray spectroscopy (EDS) was used to analyze the base material of a Gillette Fusion® razor. Results of the analysis are shown in FIG. 8A. This revealed the blade to be a form of 400 series stainless steel, however, there was some columbium observed in the base metal. This is likely used as a carbide former increasing the hardness of the blade itself. The Gillette Fusion® razor was used seven days without any treatment. FIG. 8B illustrates the blade after use and FIG. 8C is a scanning electron micrograph of the surface of the blade showing material deposited on the surface of the blade. An EDS analysis shown in FIG. 9D reveals the product deposited on the blade consisted of carbon, calcium, sulfur and oxygen.

### EXAMPLE 9

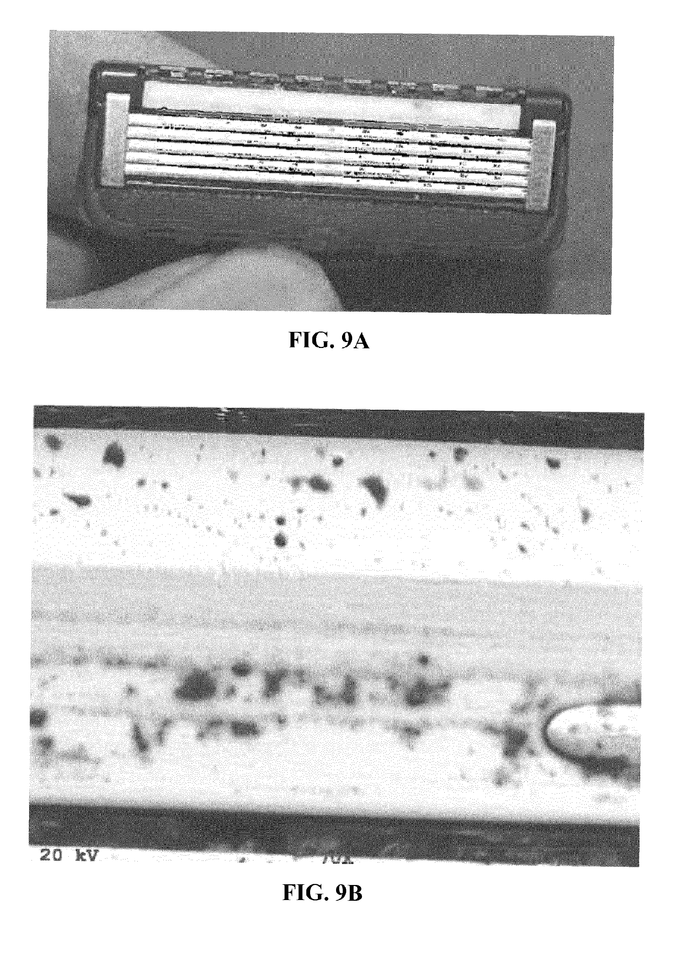

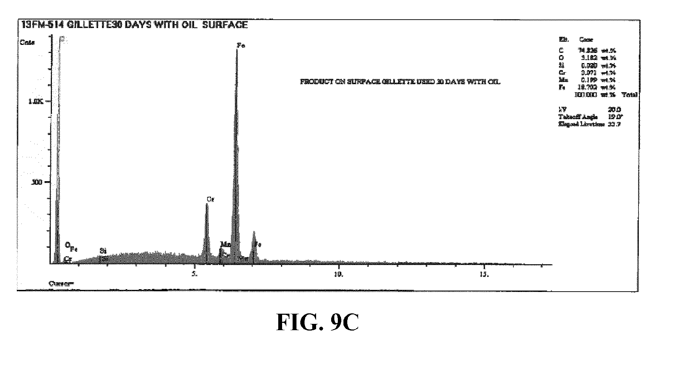

A Gillette Fusion® razor blade was treated according to the methods of the invention using the composition of EXAMPLE 1B after each use and used for 30 days. The blade is shown in FIG. 9A after 30 days of use.. This blade revealed no indications of corrosion revealing that it was at least four times more corrosion resistant than the unit without the additive. A scanning electron microscope view of the blade is shown in FIG. 9B showing very little deposition of material on the surface of the blades. The small amount of material on the blade surface was subject to EDS analysis, the results are shown in FIG. 9C. The analysis shows that the material is oil based, arising from the inventive composition, and not corrosion related. This reveals that after thirty days of use in conjunction with the composition and method of the invention there was no corrosion observed.

Nothing in this specification should be considered as limiting the scope of the present invention. All examples presented are representative and non-limiting. The above-described embodiments of the invention may be modified or varied, without departing from the invention, as appreciated by those skilled in the art in light of the above teachings. It is therefore to be understood that, within the scope of the claims and their equivalents, the invention may be practiced otherwise than as specifically described.

## Claims

1. A composition for extending the usable life of a razor blade comprising an oil, Aloe Vera gel and Petrolatum.

2. The composition of claim 1, wherein the oil is a vegetable oil

3. The composition of claim 2, wherein the vegetable oil, Aloe Vera gel and Petrolatum comprise at least about 98% of the composition.

4. The composition of claim 2, wherein the vegetable oil comprises sunflower oil.

5. The composition of any of claims 1-4, further comprising Aloe concentrate.

6. The composition of claim 5, wherein the vegetable oil, Aloe Vera gel, Petrolatum and Aloe concentrate comprise at least about 99% of the composition.

7. The composition of any one of claims 1-6, further comprising at least one of Vitamin E, Vitamin D, Vitamin A, retinyl palmitate, BHT, Tea Tree Oil and Jojoba oil.

8. The composition of claim 2, comprising about 85% to about 95% vegetable oil, about 2.5% to about 7.5% petrolatum, and about 2.5% to about 7.5% Aloe Vera Gel.

9. The composition of claim 8, wherein the vegetable oil is sunflower oil.

10. The composition of claim 9, further comprising about 0.5% to about 1.5% Aloe concentrate.

11. The composition of any one of claims 1-7, further comprising one or more components selected from: from about 0.05% to about 0.5% Vitamin E, from about 0.05% to about 0.5% Vitamin D, from about 0.05% to about 0.5% Vitamin A or retinyl palmitate, about 0.5% to about 2.0% Aloe concentrate or from about 0.01 % to about 0.1 % BHT.

12. The composition of claim 11, comprising includes about 89.65 % Sunflower Oil, about 5.00 % Petrolatum, about 5.00 % Aloe Vera Gel, about 0.10 % Vitamin-D, about 0.10 % Vitamin-E, about 0.10 % retinyl palmitate, about 1.00 % Aloe Concentrate, about 0.05 % BHT.

13. A method of extending the usable life of a razor blade comprising the step of applying the composition of any one of the preceding claims to a razor blade after use and optionally at least partially drying the razor blade before applying the composition.

14. The method of claim 13, wherein applying the composition comprises applying comprises
placing one to three drops of the composition,
dispensing the composition onto a substrate and contacting the razor blade with the substrate to transfer at least a portion of the composition to the razor blade, or
applying the composition to an applicator and contacting the applicator with the razor blade.

15. The method of claim 13, further comprising the step of using the razor blade a second time after applying the composition without rinsing or removing the composition from the razor blade.
